(19) Europäisches Patentamt
European Patent Office
Office européen des brevets

(11) Numéro de publication: **0 034 544**
**B1**

(12) # FASCICULE DE BREVET EUROPEEN

(45) Date de publication du fascicule du brevet: **18.05.83**

(51) Int. Cl.[3]: **C 07 C 148/04**, C 07 C 149/247

(21) Numéro de dépôt: **81400253.1**

(22) Date de dépôt: **18.02.81**

(54) **Nouveau procédé de préparation de la méthionine.**

(30) Priorité: **19.02.80 FR 8003581**

(43) Date de publication de la demande:
**26.08.81 Bulletin 81/34**

(45) Mention de la délivrance du brevet:
**18.05.83 Bulletin 83/20**

(84) Etats contractants désignés:
**AT BE CH DE FR GB IT LI LU NL SE**

(56) Documents cités:
**FR - A - 2 097 381**
**FR - A - 2 260 558**
**US - A - 3 330 749**

(73) Titulaire: **RHONE-POULENC SANTE**
**Les Miroirs 18 Avenue d'Alsace**
**F-92400 Courbevoie Cedex (FR)**

(72) Inventeur: **Bachot, Jean**
**11 bis, rue Rémy Laurent**
**F-92260 Fontenay aux Roses (FR)**
Inventeur: **Grosbois, Jean**
**357, Parc de Cassan**
**F-95290 L'Isle-Adam (FR)**

(74) Mandataire: **Pilard, Jacques et al,**
**RHONE-POULENC RECHERCHES Service Brevets**
**Pharma 25, Quai Paul Doumer**
**F-92408 Courbevoie Cedex (FR)**

**EP 0 034 544 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Nouveau procédé de préparation de la méthionine

La présente invention concerne un procédé de préparation de la méthionine cristallisée à partir d'une solution aqueuse contenant essentiellement un méthioninate de métal alcalin par mise en oeuvre d'un procédé d'électro-électrodialyse.

Il est connu de préparer la méthionine par déplacement d'un de ses tels, tel q'un sel de métal alcalin ou alcalino-terreux, au moyen d'un acide minéral fort tel que les acides sulfurique ou chlorhydrique. La mise en oeuvre d'un tel procédé entraîne souvent la coproduction d'une sel minéral, tel que le sulfate de sodium, difficilement exploitable ou commercialisable.

Par ailleurs, il est connu, en particulier d'après les brevets français 75 04380 (2 260 558) et américain 3 330 749 de préparer des acides organiques, tel que l'acide citrique, ou des amino-acides par application de procédés électrolytiques. Cependant, ce procédés nécessitent de nombreux recyclages des solutions de sels non hydrolysés ou bien ne peuvent pas être mis en oeuvre en continu.

La présente invention concerne un procédé de préparation en continu, de méthionine sous forme cristallisée à partir d'une solution de méthioninate alcalin contenant un électrolyte support constitué éventuellement par un carbonate du même métal alcalin par mise en oeuvre d'une cellule d'électro-électrodialyse, soumise à une différence de potentiel suffisante, qui comporte un compartiment intermédiaire, séparé de chaque compartiment contenant les électrodes par au moins une membrane cationique, dans lequel les ions de métal alcalin du méthioninate sont remplacés par les protons provenant du compartiment anodique, puis isolement de la méthionine libre sous forme cristallisée et récuperation de l'hydroxyde de métal alcalin régénéré dans le compartiment cathodique.

Le procédé d'électrodialyse selon la présente invention est réalisé dans une cellule d'électro-électrodialyse comportant trois compartiments séparés par des membranes permsélectives cationiques.

Dans le compartiment anodique, séparé du compartiment central par une membrane appelée membrane "anodique", l'oxydation de l'eau est réalisée selon la réaction:

$$H_2O-2e\rightarrow 1/2O_2\nearrow+2H^+$$

Dans le compartiment cathodique, séparé du compartiment central par une membrane appelée membrane "cathodique", la réduction de l'eau est réalisée selon la réaction:

$$2H_2O+2e\rightarrow H_2\nearrow+2OH^-$$

Les protons formés au cours de la réaction d'oxydation traversant la membrane anodique par électromigration et assurent ainsi le déplacement du sel du composé à fonction acide contenu dans le compartiment central.

Simultanément, les ions alcalins libérés traversent la membrane cathodique et se combinent dans le compartiment cathodique avec les anions hydroxy pour donner l'hydroxyde alcalin correspondant.

La méthionine est séparée de la solution provenant du compartiment central par les méthodes appropriées, par exemple séparation par filtration, des cristaux formés après refroidissement.

L'anolyte contenu dans le compartiment anodique est constitué par une solution aqueuse d'un acide minéral fort. L'anion de l'acide minéral fort doit être tel qu'il soit inerte électrochimiquement. A cet effet, les acides sulfurique ou nitrique conviennent particulièrement bien. La concentration en acide dans le compartiment anodique est généralement comprise entre 1 et 4 N et de préférence entre 1 et 2 N et elle est fonction des caractéristiques des électrodes.

Cependant il est possible que la concentration en acide soit plus faible et puisse atteindre 0,1 N si le fonctionnement de l'électrode s'en trouve amélioré. La perte d'eau due à l'électrolyse est compensée par une addition continue d'eau dans la cellule.

Le catholyte est constitué d'une solution d'un hydroxyde alcalin dont la concentration est généralement comprise entre 2 et 14 N et de préférence entre 6 et 12 N. La concentration dépend essentiellement de la qualité de la membrane. L'hydroxyde alcalin formé est soutiré en continu et sa concentration dans la cellule est maintenue constante par une addition d'eau en continu.

Dans le compartiment central, dont l'épaisseur est généralement comprise entre 1 et 3 cm mais peut être inférieure et voisine de 0,5 cm, se trouve une solution aqueuse d'un sel alcalin de la méthionine contenant un électrolyte support. L'électrolyte support peut être un sel minéral fortement dissocié, très soluble dans le milieu, dont la conductibilité est élevée, un carbonate du même métal alcalin ou leur mélange. L'électrolyte support doit être choisi également de telle manière que la séparation ultérieure de la méthionine cristallisée ne soit pas gênée par sa présence.

Le déplacement de la méthionine dans le compartiment central se fait généralement à un pH aussi voisin que possible de celui de son point isoélectrique ou de son point de neutralisation. Le pH est généralement compris entre 2 et 6 et de préférence voisin de 4 lorsque l'électrolyte support contient un

sel minéral fortement dissocié. Le pH peut être régulé automatiquement et/ou par addition d'un sel pouvant tamponner le milieu, tel que l'acétate de sodium ou le phosphate de sodium.

Lorsque l'électrolyte support est constitué uniquement par un carbonate alcalin, il est particulièrement avantageux de travailler à un pH plus élevé de façon qu'il n'y ait qu'un déplacement partiel du sel alcalin considéré, c'est-à-dire à un pH compris entre 6 et 10 et plus particulièrement entre 8 et 9.

Lorsque l'anolyte est constitué d'acide sulfurique l'anode est généralement constituée d'une plaque de titane recouverte de bioxyde de plomb ou de platine galvanique ou d'une plaque de titane recouverte de métaux précieux ou d'oxydes de métaux précieux ou d'oxyge de manganèse. De préférence l'anode est en titane recouvert de platine galvanique. Il est également possible d'utiliser avantageusement une grille de platine.

La cathode est généralement en nickel ou en fer doux éventuellement recouvert d'un revêtement catalytique.

Les membranes doivent répondere à certaines caractéristiques. Plus particulièrement elles doivent être cationiques, résister au milieu et être très conductrices.

La membrane anodique doit avoir un nombre de transport de cations aussi proche que possible de 1 et elle doit être imperméable à l'anion.

La membrane cathodique ne doit transporter que des cations communs avec ceux de l'hydroxyde produit à la cathode et elle doit avoir des performances élevées.

Les résines échangeuses d'ions qui peuvent être utilisées pour fabriquer les membranes sont les résines sulfoniques échangeuses de cations et les résines carboxyliques échangeuses de cations. Dans les premières, les groupes échangeurs d'ions sont des radicaux acides sulfoniques hydratés ($SO_3H$, $xH_2O$) et dans les secondes, ce sont des groupes carboxyliques (COOH). De préférence, le membranes en polymère sulfoné conviennent particulièrement bien. Parmi celles-ci, les membranes d'acides sulfoniques perfluorocarbonés assurent un excellent transport des cations, sont très stables, ne sont pas attaquées par les acides et les oxydants forts et présentent une excellente stabilité thermique. Les membranes commercialisées par la Société Du Pont de Nemours sous la marque Nafion conviennent particulièrement bien.

La tension aux bornes de la cellule, compte tenu de la surtension de chacune des deux électrodes, de la résistance des membranes échangeuses d'ions et des réactions secondaires pouvant se produire aux électrodes et de la distance séparant les électrodes, est généralement comprise entre 4 et 10 volts.

Il est souhaitable de travailler à de hautes densités de courant généralement voisine de 25 à 30 A/dm2, ce qui demande moins de cellules et de plus faibles dépenses d'investissement.

Il est particulièrement avantageux d'opérer à une température comprise entre 75 et 95°C et de préférence voisine de 90°C.

La figure 1 donne une schéma de réalisation de la cellule, dans le cas où la méthionine est sous forme de sel de sodium.

Par exemple, un procédé de préparation de la méthionine par voie chimique à partir de l'aldéhyde méthylthio-3 propionique peut être schématisé de la façon suivante:

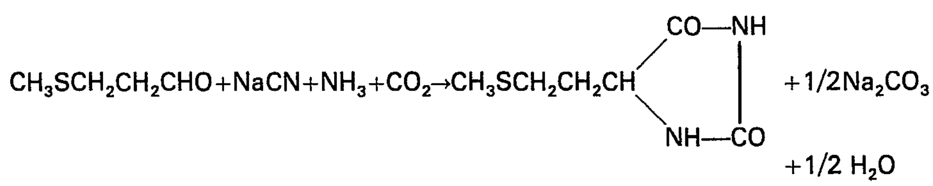

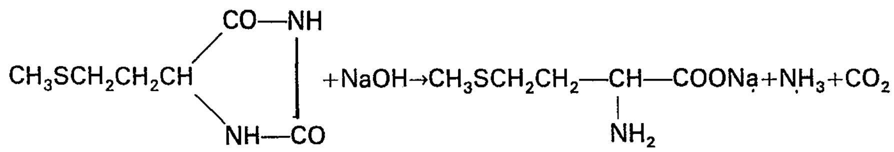

méthioninate de sodium

D'autres procédés utilisent la potasse à la place de la soude ou passent intermédiairement par un aminonitrile.

Par voie chimique, la méthionine peut être déplacée de son sel de sodium ou de potassium par action de l'acide sulfurique sur le mélange réactionnel qui contient, outre le méthioninate de sodium ou de potassium du carbonate de sodium ou de potassium.

En utilisant le procédé électrolytique décrit ci-dessus il est, par exemple, possible de déplacer la méthionine de son sel de sodium ou de potassium en évitant la production de sulfate de sodium ou de potassium. La soude ou la potasse étant régénérée dans le compartiment cathodique, celle-ci peut être réutilisée pour l'hydrolyse de l'hydantoïne ou de l'aminonitrile.

Par ailleurs, lorsque le compartiment central est alimenté par une solution contenant un mélange

de méthioninate et de carbonate alcalin, le gaz carbonique produit dans le compartiment central peut être récupéré et réutilisé dans la mise en oeuvre du procédé chimique.

Dans la cellule d'électro-électrodialyse,

— le compartiment anodique contient une solution d'acide fort, tel que l'acide sulfurique ou nitrique dont la concentration est généralement comprise entre 2 et 5 N mais peut descendre à la limite jusqu'à 0,1 N et il est alimenté en eau,
— le compartiment cathodique contient une solution aqueuse de soude ou de potasse qui s'enrichira progressivement en soude ou potasse. Lorsque la concentration en soude ou en potasse atteint le degré souhaité (2 à 14 N), la solution d'hydroxyde alcalin est soutirée et la cellule est alimentée en eau au même débit, de façon à maintenir la concentration constante au sein du compartiment cathodique.
— le compartiment central est alimenté par une solution de méthioninate de sodium ou de potassium contenant du carbonate de sodium ou de potassium. L'électrolyte support peut être constitué par un sulfate ou un nitrate alcalin ou par du carbonate de potassium. Simultanément au déplacement de la méthionine de son sel, il y a déplacement du gaz carbonique du carbonate de sodium ou de potassium. La présence du gaz carbonique étant néfaste dans la cellule (augmentation de la tension à cause de la résistance des bulles), le déplacement peut être effectué hors de la cellule les protons étant transférés dans un compartiment, indépendent de l'électrolyseur, qui contient la solution du méthioninate alcalin et du carbonate alcalin.

L'anode, la cathode et les membranes utilisées sont du type décrit ci-dessus.

La tension aux bornes de la cellule est généralement voisine de 6 volts et la densité de courant est voisine de 25 A/dm2.

La température au sein de l'électrolyseur est voisine de 80°C.

La méthionine est séparée du compartiment où s'effectue le déplacement de son sel selon les méthodes classiques. La méthionine précipite de sa solution et est séparée par filtration. Certains additifs connus pour faciliter la cristallisation peuvent être ajoutés tels que des alcools phénols, dérivés solubles de la cellulose,... Le filtrat qui contient l'électrolyte est recyclé dans le compartiment central.

La figure 2 donne le schéma du fonctionnement de la cellule pour l'isolement de la méthionine et la figure 3 donne le schéma de fonctionnement lorsque la méthionine est déplacée dans un compartiment indépendant de l'électrolyseur.

Les exemples suivants illustrant la mise en oeuvre du procédé.

Exemple 1

La cellule comporte trois compartiments séparés par des membranes sulfoniques commercialisées par la Société Du Pont de Nemours sous la marque Nafion. La membrane séparant le compartiment central du compartiment anodique est une membrane Nafion 110 et celle séparant le compartiment central du compartiment cathodique est une membrane Nafion 215.

L'anode est constituée d'une grille en métal déployé (titane) de 1 m2 recouvert d'oxyde de plomb ($PbO_2$) et la cathode est constituée d'une grille en acier gantois de même surface recouvert d'un revêtement électrocatalytique à base de titane et de nickel.

L'anolyte est constitué d'une solution d'acide sulfurique 2 N.

Le compartiment cathodique est alimenté en eau de telle sorte que la concentration en soude formée à la cathode atteigne environ 26% en poids.

Le compartiment central d'épaisseur 26 mm, est alimenté par une solution de méthioninate de sodium et de carbonate de sodium et par une solution de sulfate de sodium (de telle manière que la concentration en sulfate de sodium dans le mélange soit 2M) destinée à augmenter la conductibilité électrique du mélange. (La solution de sulfate de sodium provient de la séparation ultérieure de la méthionine).

Le débit de la solution de méthionate de sodium et de carbonate de sodium (qui contient environ 1 mole/litre de méthioninate et 0,85 mole/litre de carbonate de sodium) est de l'ordre de 30 litres/heure et est fonction du pH du compartiment central qui est réglé au voisinage de 5. (Ce pH correspond à la formation quantitative de méthionine et d'anhydride carbonique).

La cellule travaillant sous une densité de courant de 25 A/dm2, la tension s'établit entre 6,5 et 7 volts.

En opérant dans ces conditions, la production de méthionine est voisine de 4,5 kg/heure et celle de soude de 13 kg/heure (solution à 26% en poids). Le rendement faradique est de l'ordre de 90%.

Le mélange issu du compartiment central est ensuite soutiré et la séparation de la méthionine du sulfate de sodium est effectuée selon les procédés habituels: refroidissement à une température voisine de 40°C et séparation de la méthionine cristallisée par filtration. Le filtrat, qui contient du sulfate de sodium est recyclé dans le compartiment central.

Exemple 2

On utilise une cellule identique à celle qui est décrite dans l'exemple 1 dans laquelle la membrane

Nafion 110 est remplacée par une membrane Nafion 117 et dans laquelle le compartiment central a une épaisseur de 13 mm, l'anode, la cathode et l'anolyte étant les mêmes que dans l'exemple 1 et le compartiment cathodique étant alimenté en eau de la même manière.

Le compartiment central est alimenté par une solution de méthioninate de sodium et de carbonate de sodium et par une solution de sulfate de sodium, de telle manière que la concentration en sulfate de sodium dans le mélange soit 1 M.

Le débit de la solution de méthioninate de sodium et de carbonate de sodium (qui contient environ 1 mole/litre de méthioninate et 0,85 mole/litre de carbonate de sodium) est de l'ordre de 30 litres/heure et est fonction du pH du compartiment central qui est réglé au voisinage de 5. Dans ces conditions, la concentration réelle dans le compartiment central, compte tenu du débit de la solution de sulfate de sodium, est voisine de 0,5 mole/litre en méthioninate de sodium et 0,43 mole/litre en carbonate de sodium.

La cellule travaillant sous une densité de courant de 25 A/dm$^2$, la tension s'établit à 6,4 volts.

En opérant dans ces conditions la production de méthionine est voisine de 4,5 kg/heure et celle de soude de 13 kg/heure (solution à 26% en poids). Le rendement faradique est de l'ordre de 90%.

La méthionine est isolée dans les conditions décrites à l'exemple 1.

Exemple 3

On utilise une cellule identique à celle qui est décrite dans l'exemple 1.

L'anode est constituée d'une grille de titane déployé de 1 m2 recouvert de platine déposé électrochimiquement (épaisseur: 10 $\mu$ environ) et la cathode est constituée d'une grille en acier gantois de même surface.

L'anolyte est constitué d'une solution d'acide nitrique 2 N.

Le compartiment cathodique est alimenté en eau de telle sorte que la concentration en soude formée à la cathode atteigne environ 26% en poids.

Le compartiment central d'épaisseur 26 mm est alimenté par une solution de méthioninate de sodium et de carbonate de sodium contenant du nitrate de sodium (en quantité telle que sa concentration dans le mélange soit environ 5 N) pour augmenter la conductibilité électrique du mélange.

Le débit de la solution de méthioninate de sodium et de carbonate de sodium (qui contient environ 1 mole/litre de méthioninate et 0,85 mole/litre de carbonate de sodium) est de l'ordre de 30 litres/heure et est fonction du pH du compartiment central qui est réglé au voisinage de 5.

La cellule travaillant sous une densité de courant de 25 A/dm2, la tension s'établit vers 6 volts.

En opérant dans ces conditions, la production de méthionine est voisine de 4,5 kg/heure et celle de soude de 13 kg/heure (solution à 26% en poids). Le rendement faradique est de l'ordre de 90%.

La méthionine est isolée dans les conditions décrites à l'exemple 1.

Exemple 4

On utilise une cellule identique à celle qui est décrite dans l'exemple 1.

Cependant l'anode est constituée d'un grille fine de platine (220 g/m$^2$) et le compartiment central a une épaisseur de 13 mm.

L'anolyte est constitué d'une solution d'acide nitrique 2 N.

Le compartiment cathodique est alimenté en eau de telle sorte que la concentration en soude formée à la cathode atteigne environ 26% en poids.

Le compartiment central est alimenté par une solution de méthioninate de sodium et de carbonate de sodium et par une solution de nitrate de sodium (en quantité telle que la concentration en nitrate de sodium dans le mélange soit environ 3 N).

Le débit de la solution de méthioninate de sodium et de carbonate de sodium (qui contient environ 1 mole/litre de méthioninate et 0,85 mole/litre de carbonate de sodium) est de l'ordre de 30 litres/heure et varie en fonction de la variation du pH ajusté initialement au voisinage de 5.

La cellule travaillant sous une densité de courant de 25 A/dm$^2$, la tension s'établit à 5,5 volts.

En opérant dans ces conditions, la production de méthionine est voisine de 4,5 kg/heure et celle de soude de 13 kg/heure (solution à 26% en poids). Le rendement faradique est de l'ordre de 90%.

La méthionine est isolée dans les conditions décrites à l'exemple 1.

Exemple 5

On utilise une cellule identique à celle qui est décrite dans l'exemple 1. L'anode est constituée d'une grille en métal déployé (titane) de 1 m2 recouvert d'oxyde de plomb ($PbO_2$) et la cathode est constituée d'une grille en acier gantois.

L'anolyte est constitué d'une solution d'acide sulfurique 2 N.

Le compartiment cathodique est alimenté en eau de telle sorte que la concentration en potasse formée à la cathode atteigne 26% en poids.

Le compartiment central d'épaisseur 26 mm, est alimenté par une solution de méthioninate de potassium et de carbonate de potassium. Il n'est pas ajouté d'électrolyte support.

Le pH est fixé à une valeur telle que le taux de transformation global soit d'environ 50% c'est-à-dire entre pH 10,2 et pH 6,4 et de préférence au voisinage de 8.

Pour favoriser la stabilité du carbonate acide de potassium qui assure le passage du courant, et retarder sa complète neutralisation, il est possible de se placer sous une légère pression.

Le débit de la solution de méthioninate de potassium et de carbonate de potassium est de l'ordre de 30 litres/heure et est fonction du pH du compartiment central qui est réglé au voisinage de 8.

La cellule travaillant sous une densité de courant de 25 A/dm2 la tension s'établit vers 8,5 volts.

En opérant dans ces conditions, la production de méthionine est voisine de 4,5 kg/heure et celle de potasse de 18,0 kg/heure (solution à 26% en poids). Le rendement faradique est de l'ordre de 90%.

La méthionine est isolée dans les conditions décrites à l'exemple 1.

Exemple 6

On utilise une cellule identique à celle qui est décrite dans l'exemple 1.

Cependant, la membrane cathodique est une membrane Nafion 214 et l'anode est une grille fine de platine (220 g/m2).

L'anolyte est constitué d'une solution d'acide sulfurique 0,5 N.

Le compartiment cathodique est alimenté en eau de telle sorte que la concentration en potasse formée à la cathode atteigne 26% en poids.

Le compartiment central, d'épaisseur 13 mm, est alimenté par une solution de méthioninate de potassium et de carbonate de potassium.

Le pH est ajusté à 8,7; dans ces conditions le taux de transformation est de 53%.

Le débit de la solution de méthioninate de potassium et de carbonate de potassium est réglé en fonction du pH du compartiment central.

La cellule travaillant sous une densité de courant de 25 A/dm2, la tension s'établit vers 6,5 volts.

En opérant dans ces conditions, la production de méthionine est voisine de 4,5 kg/heure et celle de potasse de 18 kg/heure (solution de 26% en poids). Le rendement faradique est de l'ordre de 90%.

La méthionine est isolée dans les conditions décrites à l'exemple 1.

Exemple 7

On opère dans les conditions décrites à l'exemple 6 en remplaçant la solution de méthioninate de potassium et de carbonate de potassium par une solution de méthioninate de sodium et de carbonate de sodium.

Le pH du compartiment central étant ajusté à 8,4, le taux de transformation est voisin de 50%.

La cellule travaillant sous une densité de courant de 25 A/dm2 la tension s'établit vers 7,1 volts.

En opérant dans ces conditions, la production de méthionine est voisine de 4,5 kg/heure et celle de soude de 13 kg/heure (solution à 26% en poids). Le rendement faradique est voisin de 90%.

La méthionine est isolée dans les conditions décrites à l'exemple 1.

**Revendications**

1. Procédé de préparation en continu de méthionine sous forme cristallisée caractérisé en ce que l'on soumet à une différence de potentiel suffisante une solution d'un méthioninate alcalin et d'un électrolyte support contenue dans le compartiment intermédiaire d'une cellule d'électro-électrodialyse, séparé de chaque compartiment contenant les électrodes par au moins une membrane cationique, ou dans un compartiment annexe de ce compartiment intermédiaire, puis isole la méthionine, formée dans le compartiment intermédiaire, sous forme cristallisée et récupère l'hydroxyde alcalin régénéré dans le compartiment cathodique.

2. Procédé selon la revendication 1 caractérisé en ce que l'électrolyte support est constitué par un sel fortement dissocié du même métal alcalin.

3. Procédé selon la revendication 1 caractérisé en ce que l'électrolyte support est constitué par un mélange d'un sel fortement dissocié et d'un carbonate du même métal alcalin.

4. Procédé selon la revendication 1 caractérisé en ce que l'électrolyte support est constitué par un carbonate alcalin.

5. Procédé selon la revendication 1 caractérisé en ce que l'anolyte contenu dans le compartiment anodique est constitué par une solution aqueuse d'un acide minéral fort choisi parmi les acides sulfurique et nitrique à une concentration comprise entre 0,1 et 5 N.

6. Procédé selon la revendication 1 caractérisé en ce que le catholyte contenu dans le compartiment cathodique est constitué par une solution d'hydroxyde alcalin dont la concentration maintenue constante est comprise entre 2 et 14 N.

7. Procédé selon la revendication 1 caractérisé en ce que l'anode est constituée d'une plaque de titane recouverte de bioxyde de plomb ou de platine galvanique ou d'un grillage fin de platine et la cathode est en fer doux ou en nickel éventuellement recouvert d'un revêtement catalytique.

8. Procédé selon la revendication 1 caractérisé en ce que le débit de la solution de méthioninate alcalin est réglé en fonction du pH du compartiment central qui est compris entre 3 et 6 lorsque l'électrolyte support contient un sel minéral fortement dissocié ou entre 7 et 10 lorsque l'électrolyte support est constitué uniquement par un carbonate du même métal alcalin.

9. Procédé selon la revendication 1 caractérisé en ce que la tension aux bornes de la cellule est comprise entre 4 et 10 volts.

**Patentansprüche**

1. Verfahren zur kontinuierlichen Herstellung von Methionin in kristallisierter Form, dadurch gekennzeichnet, daß man eine in einer von den die Elektroden enthaltenden Kammern einer Elektro-Dialysezelle durch jeweils zumindest eine kationische Membran getrennten Zwischenkammer oder in einer an die Zwischenkammer angeschlossenen Kammer enthaltene Lösung eines Alkalimethioninats und eines Elektrolytträgers unter eine ausreichende Potentialdifferenz setzt, das in der Zwischenkammer gebildete Methionin in kristallisierter Form isoliert und das in der Kathodenkammer regenierte Alkalihydroxid rückgewinnt.

2. Verfahren nach Anspruch 1, dadurch gekennzeichnet, daß der Elektrolytträger aus einem stark dissoziierten Salz des gleichen Alkalimetalls besteht.

3. Verfahren nach Anspruch 1, dadurch gekennzeichnet, daß der Elektrolytträger aus einer Mischung eines stark dissoziierten Salzes und eines Carbonats des gleichen Alkalimetalls besteht.

4. Verfahren nach Anspruch 1, dadurch gekennzeichnet, daß der Elektrolytträger aus einem Alkalicarbonat besteht.

5. Verfahren nach Anspruch 1, dadurch gekennzeichnet, daß der in der Anodenkammer enthaltene Anolyt aus einer wässerigen Lösung einer starken anorganischen Säure, ausgewählt aus Schwefelsäure und Salpetersäure, mit einer Konzentration von 0,1 bis 5n besteht.

6. Verfahren nach Anspruch 1, dadurch gekennzeichnet, daß der in der Kathodenkammer enthaltene Katholyt aus einer Alkalihydroxidlösung besteht, deren Konzentration konstant zwischen 2 und 14 n gehalten wird.

7. Verfahren nach Anspruch 1, dadurch gekennzeichnet, daß die Anode aus einer Titanplatte, die mit Bleioxid oder galvanischem Platin überzogen ist, oder aus einem Platingitter besteht und daß die Kathode aus Weicheisen oder Nickel, gegebenenfalls mit einem katalytischen Überzug, besteht.

8. Verfahren nach Anspruch 1, dadurch gekennzeichnet, daß die Zufur der Alkalimethioninatlösung in Abhängigkeit vom pH-Wert der zentralen Kammer geregelt wird, welcher zwischen 3 und 6 liegt, wenn der Elektrolytträger ein stark dissoziiertes anorganisches Salz enthält, oder welcher zwischen 7 und 10 liegt, wenn der Elektrolytträger ausschließlich aus einem Carbonat des gleichen Alkalimetalls besteht.

9. Verfahren nach Anspruch 1, dadurch gekennzeichnet, daß die Spannung an den Zellenausgängen zwischen 4 und 10 Volt liegt.

**Claims**

1. Continuous process for the preparation of methionine in the crystalline form, characterised in that a solution of an alkali metal methioninate and of a supporting electrolyte, which is contained in the intermediate compartment of an electrolysis/electrodialysis cell, separated by means of at least one cationic membrane from each compartment containing the electrodes, or in a compartment attached to this intermediate compartment, is subjected to a sufficient potential difference, the methionine formed in the intermediate compartment is then isolated in the crystalline form and the alkali metal hydroxide regenerated in the cathode compartment is recovered.

2. Process according to Claim 1, characterised in that the supporting electrolyte consists of a strongly dissociated salt of the same alkali metal.

3. Process according to Claim 1, characterised in that the supporting electrolyte consists of a mixture of a strongly dissociated salt and a carbonate of the same alkali metal.

4. Process according to Claim 1, characterised in that the supporting electrolyte consists of an alkali metal carbonate.

5. Process according to Claim 1, characterised in that the anolyte contained in the anode compartment consists of an aqueous solution of a strong mineral acid, chosen from sulphuric acid and nitric acid, at a concentration of between 0.1 and 5 N.

6. Process according to Claim 1, characterised in that the catholyte contained in the cathode compartment consists of a solution of an alkali metal hydroxide, the concentration of which is kept constant at between 2 and 14 N.

7. Process according to Claim 1, characterised in that the anode consists of a titanium plate covered with lead dioxide or with electroplated platinum, or of a fine grid of platinum, and the cathode is made of soft iron or nickel, which may be covered with a coating of catalyst.

8. Process according to Claim 1, characterised in that the flow rate of the solution of alkali metal methioninate is adjusted according to the pH of the central compartment, which is between 3 and 6 if the supporting electrolyte contains a strongly dissociated inorganic salt, or between 7 and 10 if the supporting electrolyte consists solely of a carbonate of the same alkali metal.

9. Process according to Claim 1, characterised in that the voltage at the terminals of the cell is between 4 and 10 volts.

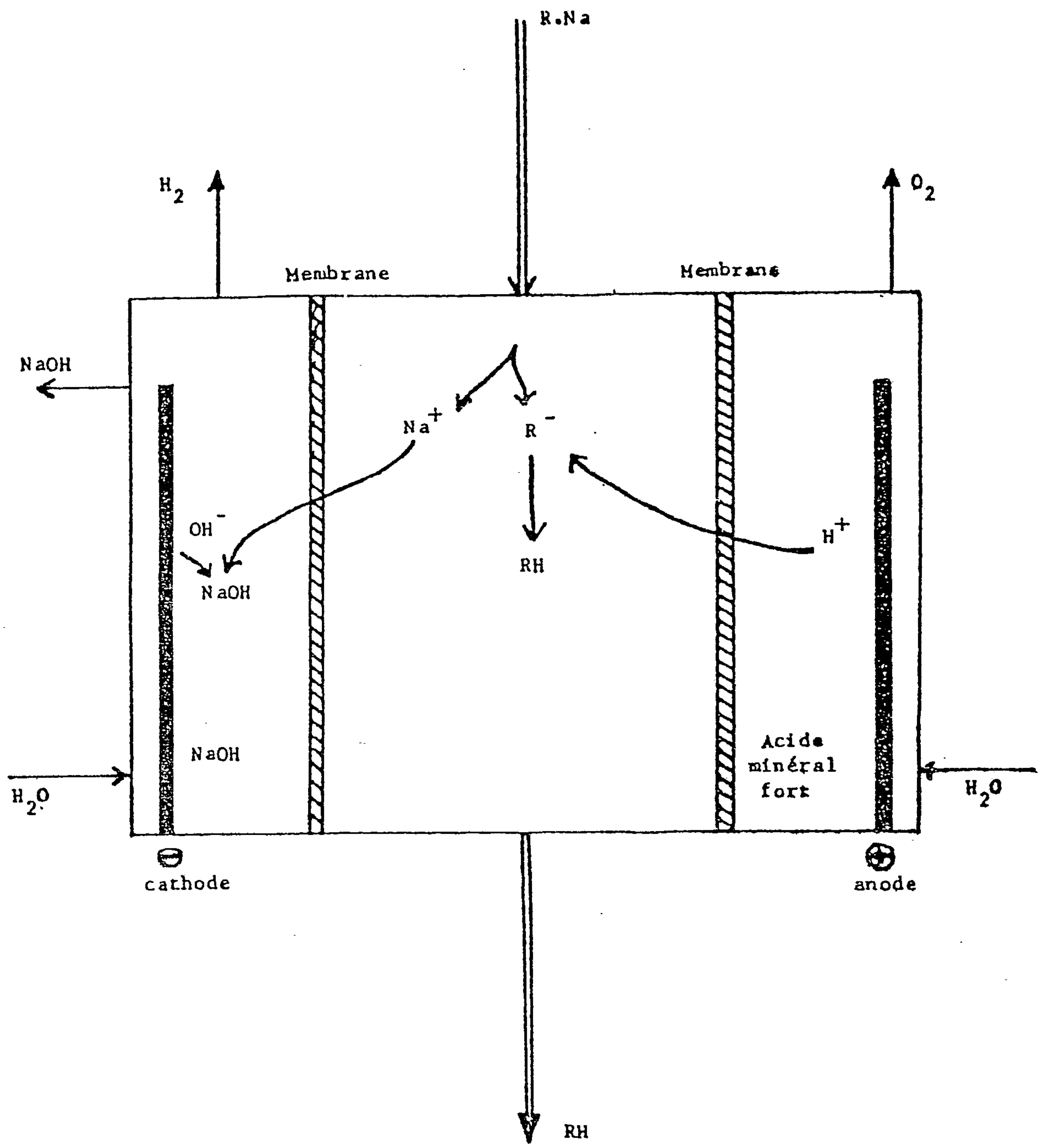


$$R = CH_3\ S\ CH_2\ CH_2 - \underset{\displaystyle NH_2}{\underset{|}{CH}} - COO$$

Figure 1

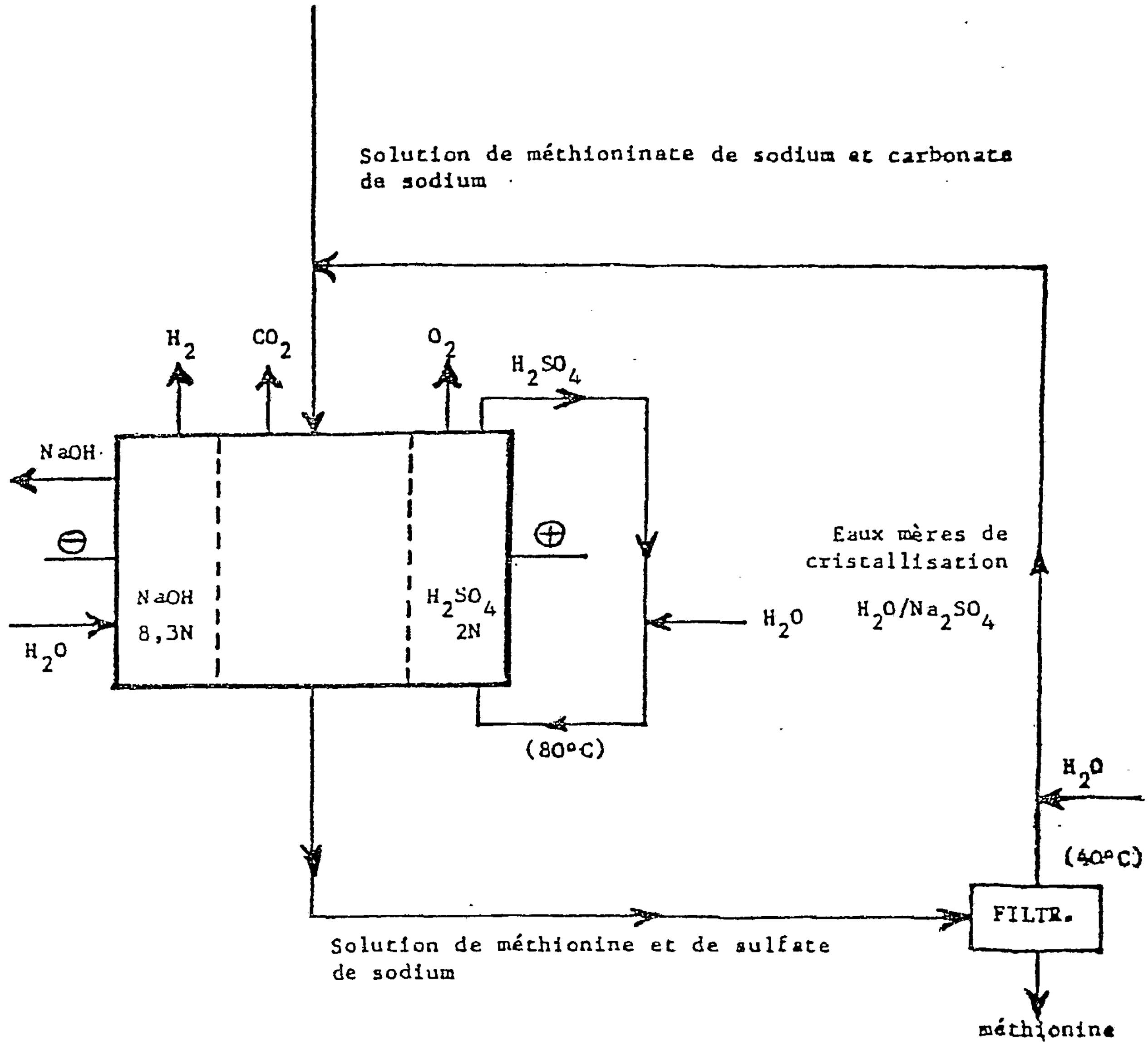
Solution de méthioninate de sodium et carbonate de sodium
H2
CO2
O2
H2SO4
NaOH
NaOH
8,3N
H2SO4
2N
H2O
H2O
Eaux mères de cristallisation
H2O/Na2SO4
(80°C)
H2O
(40°C)
FILTR.
Solution de méthionine et de sulfate de sodium
méthionine

Figure 2

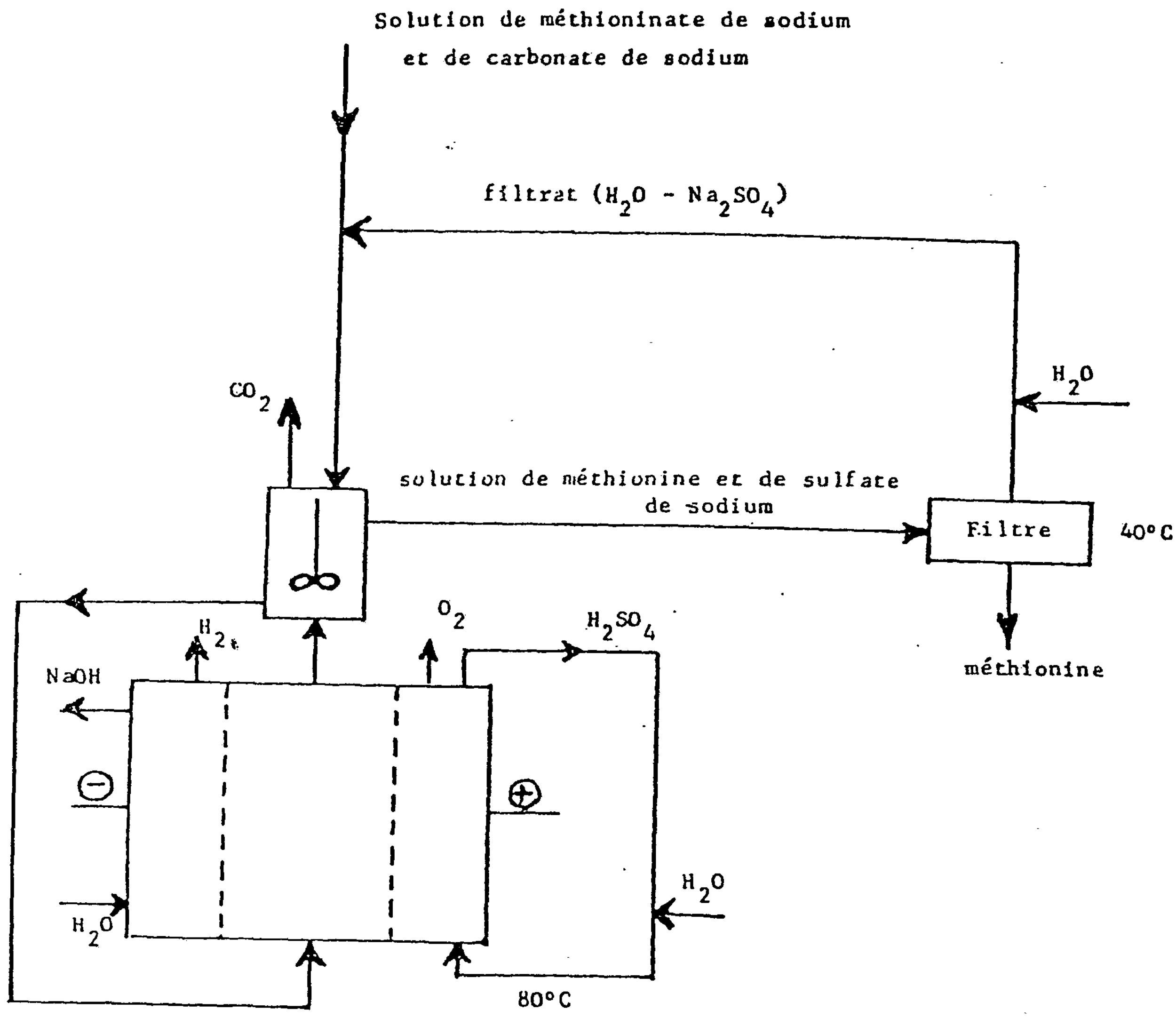
Solution de méthioninate de sodium
et de carbonate de sodium
filtrat ($H_2O$ - $Na_2SO_4$)
$CO_2$
$H_2O$
solution de méthionine et de sulfate
de sodium
Filtre
40°C
$O_2$
$H_2SO_4$
$H_2$
NaOH
méthionine
⊖
⊕
$H_2O$
$H_2O$
80°C

Figure 3